(19) Europäisches Patentamt
European Patent Office
Office européen des brevets

(11) **EP 3 627 515 A1**

(12) **EUROPEAN PATENT APPLICATION**

(43) Date of publication:
**25.03.2020 Bulletin 2020/13**

(21) Application number: **18195446.2**

(22) Date of filing: **19.09.2018**

(51) Int Cl.:
*G16H 20/30* (2018.01)　　　*G08B 21/02* (2006.01)
*G08B 21/04* (2006.01)　　　*A61H 3/00* (2006.01)
*A61B 5/11* (2006.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Koninklijke Philips N.V.
5656 AG Eindhoven (NL)**

(72) Inventors:
• **TEN KATE, Warner Rudolph Theophile
5656 AE Eindhoven (NL)**
• **SMITS, Tine
5656 AE Eindhoven (NL)**

(74) Representative: **de Haan, Poul Erik et al
Philips International B.V.
Philips Intellectual Property & Standards
High Tech Campus 5
5656 AE Eindhoven (NL)**

(54) **MONITORING A USER**

(57)　According to an aspect, there is provided a monitoring apparatus (2) for monitoring a user. The monitoring apparatus (2) comprises a processing unit (10) that is configured to receive first movement measurements from a first movement sensor (16) that is associated with the user, the first movement measurements representing movements of the user; and receive second movement measurements from a second movement sensor (20) that is associated with a first assistive technology, AT, device (8), the second movement measurements representing movements of the first AT device (8). The processing unit (10) is further configured to process the first movement measurements to determine if the user is walking; determine a correlation measure for the first movement measurements and the second movement measurements if it is determined that the user is walking, with the correlation measure representing the correlation between the movements of the user while the user is walking and the movements of the first AT device (8); and initiate an alert to the user to use an AT device if the determined correlation measure indicates that the movements of the user and the movements of the first AT device (8) are not correlated.

EP 3 627 515 A1

**Description**

FIELD OF THE INVENTION

The invention relates to a method, a computer program product and a monitoring apparatus for monitoring a user.

BACKGROUND OF THE INVENTION

Disability and mobility problems increase with age. Many community-dwelling adults (particularly those over the age of 65) use assistive technology (AT) devices to assist or improve their mobility (e.g. walking). Such AT devices include canes (otherwise known as walking sticks), walkers (also known as walking frames), customised shoes, exoskeletons and crutches. With the increasing number of older adults in the community that have one or more chronic conditions, disability and the resulting mobility problems will become more widespread. It is estimated that, of adults older than 65 years, around 10% use canes and 4.6% use walkers.

Assistive technology devices such as canes, crutches, and walkers can be used to increase a user's base of support, to improve balance, and thereby increase activity and independence. These AT devices can also add a level of safety for the user and prevent potential fall incidents. However, many users may not always remember to take their AT device with them when going for a walk, or when moving around the house.

US 2017/154514 describes a device that is to be used with a walker, with one component of the device attached to the walker and another component of the device worn by the user, with the device signalling the user of the walker to use the walker by providing an alert whenever the user moves more than a predetermined distance from the walker. The two components of the device wirelessly communicate with each other. The distance between the two components of the device can be determined by GPS technology and/or one or more proximity sensors.

SUMMARY OF THE INVENTION

A disadvantage to the existing devices is that the use of radio or similar technologies (ultrasound (US), visible light (e.g. a laser), infrared (IR) light) requires relatively complex electronics for the purpose of enabling the reminder function. The technology can also be power consuming, implying a burden on the user to recharge the device or replace a battery.

It is an object to provide an improved way of monitoring a user and providing an alert to a user that they should use an AT device.

According to a first specific aspect, there is provided a monitoring apparatus for monitoring a user. The monitoring apparatus comprises a processing unit configured to: receive first movement measurements from a first movement sensor that is associated with the user, the first movement measurements representing movements of the user; receive second movement measurements from a second movement sensor that is associated with a first assistive technology, AT, device, the second movement measurements representing movements of the first AT device; process the first movement measurements to determine if the user is walking; if it is determined that the user is walking, determine a correlation measure for the first movement measurements and the second movement measurements, wherein the correlation measure represents the correlation between the movements of the user while the user is walking and the movements of the first AT device; and initiate an alert to the user to use an AT device if the determined correlation measure indicates that the movements of the user and the movements of the first AT device are not correlated. Thus, an alert that the user should use an AT device is initiated based on a measure of the correlation between movements of the user and the first AT device. This avoids the need to use radio or similar technologies to measure the distance between the user and the AT device, and thus provides a generally lower power and lower complexity apparatus.

In some embodiments, the processing unit is further configured to update a state indication for the user and the first AT device based on the determined correlation measure, wherein the state indication has one of at least two states, wherein the at least two states comprises a near state and a distant state, wherein the state indication is set to the near state if the determined correlation measure indicates that the movements of the user and the movements of the first AT device are correlated, and wherein the state indication is set to the distant state if the determined correlation measure indicates that the movements of the user and the movements of the first AT device are not correlated.

In some embodiments, the processing unit is configured to initiate the alert to the user if the determined correlation measure indicates that the movements of the user and the movements of the first AT device are not correlated, and, before updating the state indication based on the determined correlation measure, the state indication is the near state. These embodiments help to reduce the occurrence of alerts when the user is not near to the AT device (and thus not easily able to start using the AT device).

In alternative embodiments, the processing unit is configured initiate the alert to the user if the determined correlation measure indicates that the movements of the user and the movements of the first AT device are not correlated and the state indication is updated to the distant state. These embodiments also help to reduce the occurrence of alerts when the user is not near to the AT device (and thus not easily able to start using the AT device).

In some embodiments, the processing unit is further configured to, if the determined correlation measure indicates that the movements of the user and the movements of the first AT device are not correlated, wait for the expiry of a timer before initiating the alert. These embodiments allow time for the user to start using the first AT device before the alert is initiated (and if so, the alert can be suppressed).

In some embodiments, the processing unit is configured to receive the second movement measurements from the second movement sensor after determining that the user is walking. These embodiments can reduce the power consumption of the monitoring apparatus as it is not necessary for the monitoring apparatus to receive the second movement measurements until they are required for evaluation.

In some embodiments, the processing unit is further configured to receive third movement measurements from a third movement sensor that is associated with a second AT device, the third movement measurements representing movements of the second AT device; if it is determined that the user is walking, determine a second correlation measure for the first movement measurements and the third movement measurements, wherein the second correlation measure represents the correlation between the movements of the user while the user is walking and the movements of the second AT device; and the processing unit is configured to initiate the alert to the user if the correlation measure indicates that the movements of the user and the movements of the first AT device are not correlated and the second correlation measure indicates that the movements of the user and the movements of the second AT device are not correlated. These embodiments have the advantage that multiple AT devices can be monitored, and an alert initiated only if the user is not using any of them.

In some embodiments, the monitoring apparatus further comprises the first movement sensor. In other embodiments, the first movement sensor is not part of the monitoring apparatus.

According to a second aspect, there is provided a system for monitoring a user that comprises a monitoring apparatus according to the first aspect or any embodiment thereof.

In some embodiments, the system further comprises the first movement sensor.

In some embodiments, the system further comprises the second movement sensor. In some embodiments, the second movement sensor is comprised in a first monitoring device that is associated with the first AT device.

According to a third aspect, there is provided a method of monitoring a user, the method comprising receiving first movement measurements from a first movement sensor that is associated with the user, the first movement measurements representing movements of the user; receiving second movement measurements from a second movement sensor that is associated with a first assistive technology, AT, device, the second movement measurements representing movements of the first AT device; processing the first movement measurements to determine if the user is walking; if it is determined that the user is walking, determining a correlation measure for the first movement measurements and the second movement measurements, wherein the correlation measure represents the correlation between the movements of the user while the user is walking and the movements of the first AT device; and initiating an alert to the user to use an AT device if the determined correlation measure indicates that the movements of the user and the movements of the first AT device are not correlated. Thus, an alert that the user should use an AT device is initiated based on a measure of the correlation between movements of the user and the first AT device. This avoids the need to use radio or similar technologies to measure the distance between the user and the AT device, and thus provides a generally lower power and lower complexity method.

In some embodiments, the method further comprises updating a state indication for the user and the first AT device based on the determined correlation measure, wherein the state indication has one of at least two states, wherein the at least two states comprises a near state and a distant state, wherein the state indication is set to the near state if the determined correlation measure indicates that the movements of the user and the movements of the first AT device are correlated, and wherein the state indication is set to the distant state if the determined correlation measure indicates that the movements of the user and the movements of the first AT device are not correlated.

In some embodiments, the method comprises initiating the alert to the user if the determined correlation measure indicates that the movements of the user and the movements of the first AT device are not correlated, and, before updating the state indication based on the determined correlation measure, the state indication is the near state. These embodiments help to reduce the occurrence of alerts when the user is not near to the AT device (and thus not easily able to start using the AT device).

In alternative embodiments, the method comprises initiating the alert to the user if the determined correlation measure indicates that the movements of the user and the movements of the first AT device are not correlated and the state indication is updated to the distant state. These embodiments also help to reduce the occurrence of alerts when the user is not near to the AT device (and thus not easily able to start using the AT device).

In some embodiments, the method further comprises, if the determined correlation measure indicates that the movements of the user and the movements of the first AT device are not correlated, waiting for the expiry of a timer before initiating the alert. These embodiments allow time for the user to start using the first AT device before the alert is initiated.

In some embodiments, the step of receiving the second movement measurements from the second movement sensor is performed after determining that the user is walking. These embodiments can reduce the power consumption of the method as it is not necessary to receive the second movement measurements until they are required for evaluation.

In some embodiments, the method further comprises receiving third movement measurements from a third movement sensor that is associated with a second AT device, the third movement measurements representing movements of the second AT device; if it is determined that the user is walking, determining a second correlation measure for the first movement measurements and the third movement measurements, wherein the second correlation measure represents the correlation between the movements of the user while the user is walking and the movements of the second AT device; and the step of initiating the alert to the user comprises initiating the alert to the user if the correlation measure indicates that the movements of the user and the movements of the first AT device are not correlated and the second correlation measure indicates that the movements of the user and the movements of the second AT device are not correlated. These embodiments have the advantage that multiple AT devices can be monitored, and an alert initiated only if the user is not using any of them.

According to a fourth aspect, there is provided a computer program product comprising a computer readable medium having computer readable code embodied therein, the computer readable code being configured such that, on execution by a suitable computer or processor, the computer or processor is caused to perform the method according to the third aspect, or any embodiment thereof.

These and other aspects will be apparent from and elucidated with reference to the embodiment(s) described hereinafter.

BRIEF DESCRIPTION OF THE DRAWINGS

Exemplary embodiments will now be described, by way of example only, with reference to the following drawings, in which:

Fig. 1 is a block diagram illustrating a system comprising a monitoring apparatus according to an exemplary embodiment;
Fig. 2 shows a state model according to an embodiment;
Fig. 3 shows a state model according to another embodiment;
Fig. 4 is a flow chart illustrating a method according to an exemplary embodiment;
Fig. 5 shows a first set of graphs illustrating exemplary movement measurements for a user, exemplary movement measurements for an AT device and a measure of the correlation between the movement measurements;
Fig. 6 shows a second set of graphs illustrating exemplary movement measurements for a user, exemplary movement measurements for an AT device and a measure of the correlation between the movement measurements; and
Fig. 7 shows a third set of graphs illustrating exemplary movement measurements for a user, exemplary movement measurements for an AT device and a measure of the correlation between the movement measurements.

DETAILED DESCRIPTION OF EMBODIMENTS

As noted above, embodiments described herein provide that measurements of the movements of the user and movements of an assistive technology (AT) device are collected, and these measurements are processed to determine if the user is using the AT device while the user is walking. If the processing of the measurements indicates that the user is walking but not using the AT device, an alert is initiated to remind or inform the user that they should use an AT device.

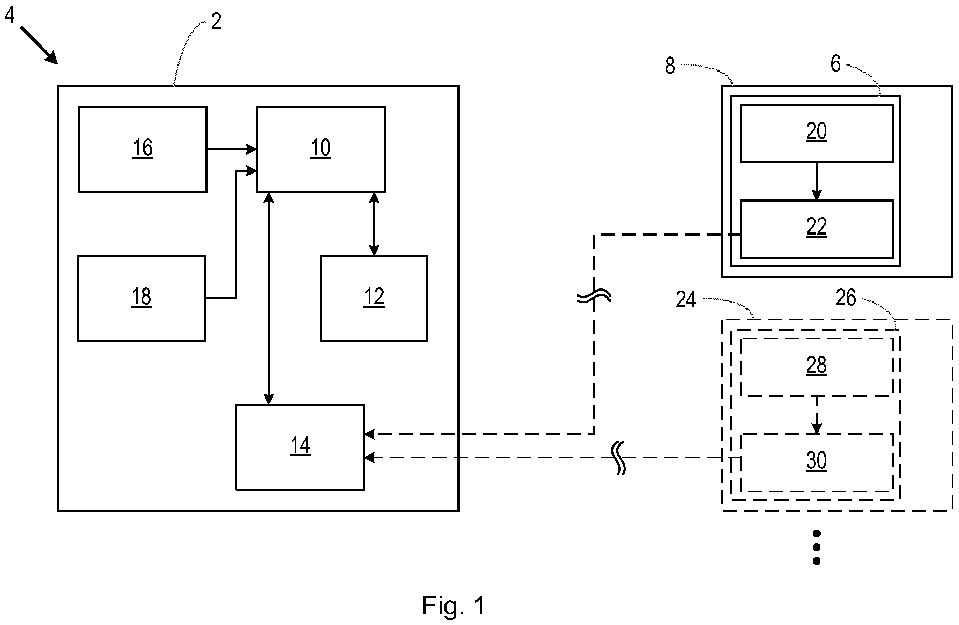

Fig. 1 illustrates a monitoring apparatus 2 according to an embodiment. The monitoring apparatus 2 is shown as part of a system 4 that also includes a first monitoring device 6. The first monitoring device 6 is associated with a first AT device 8, and the first monitoring device 6 is used to measure the movements of the first AT device 8. The first AT device 8 can be any type of device that can assist a user with their mobility, particularly walking. As such, the first AT device 8 could be a cane/walking stick, a walker/walking frame, a crutch or a part of crutches. The first monitoring device 6 may be integral to or with the first AT device 8 (e.g. the first monitoring device 6 may be inside a housing or structure of the first AT device 8), or the first monitoring device 6 may be attached or coupled to the first AT device 8 (e.g. the first monitoring device 6 may be affixed or adhered to the housing or structure of the first AT device 8). The first monitoring device 6 may be removably or permanently attached or coupled to the first AT device 8.

The monitoring apparatus 2 includes a processing unit 10 that controls the operation of the monitoring apparatus 2 and that can be configured to execute or perform the methods described herein. In particular, the processing unit 10 is provided to analyse or process measurements of the movements of a user and the first AT device 8 to determine whether an alert (reminder) should be initiated to the user to advise that they should use an AT device. The processing unit 10 can be implemented in numerous ways, with software and/or hardware, to perform the various functions described herein. The processing unit 10 may comprise one or more microprocessors or digital signal processor (DSPs) that may be programmed using software or computer program code to perform the required functions and/or to control components of the processing unit 10 to effect the required functions. The processing unit 10 may be implemented as a combination of dedicated hardware to perform some functions (e.g. amplifiers, pre-amplifiers, analog-to-digital convertors (ADCs) and/or digital-to-analog convertors (DACs)) and a processor (e.g., one or more programmed microprocessors, controllers, DSPs and associated circuitry) to perform other functions. Examples of components that may be employed in various embodiments of the present disclosure include, but are not limited to, conventional microprocessors, DSPs, application specific integrated circuits (ASICs), and field-programmable gate arrays (FPGAs).

The processing unit 10 is connected to a memory unit 12 that can store data, information and/or signals for use by the processing unit 10 in controlling the operation of the monitoring apparatus 2 and/or in executing or performing the methods described herein. In some implementations the memory unit 12 stores computer-readable code that can be executed by the processing unit 10 so that the processing unit 10 performs one or more functions, including the methods described herein. The memory unit 12 can also store measurements or measurement signals received from one or more sensors ready for subsequent processing by the processing unit 10, and/or any other information required for or during the methods and techniques described herein. The memory unit 12 can comprise any type of non-transitory machine-readable medium, such as cache or system memory including volatile and non-volatile computer memory such as random access memory (RAM) static RAM (SRAM), dynamic RAM (DRAM), read-only memory (ROM), programmable ROM (PROM), erasable PROM (EPROM) and electrically erasable PROM (EEPROM), implemented in the form of a memory chip, an optical disk (such as a compact disc (CD), a digital versatile disc (DVD) or a Blu-Ray disc), a hard disk, a tape storage solution, or a solid state device, including a memory stick, a solid state drive (SSD), a memory card, etc.

The monitoring apparatus 2 also includes interface circuitry 14 for enabling a data connection to and/or data exchange with other devices, including any one or more of servers, databases, and sensors, and particularly with monitoring devices associated with AT devices (e.g. first monitoring device 6). The connection may be direct or indirect (e.g. via the Internet), and thus the interface circuitry 14 can enable a connection between the monitoring apparatus 2 and a network, such as the Internet, via any desirable wired or wireless communication protocol. For example, the interface circuitry 14 can operate using WiFi, Bluetooth, ZigBee, or any cellular communication protocol (including but not limited to Global System for Mobile Communications (GSM), Universal Mobile Telecommunications System (UMTS), Long Term Evolution (LTE), LTE-Advanced, etc.). In the case of a wireless connection, the interface circuitry 14 (and thus monitoring apparatus 2) may include one or more suitable antennas for transmitting/receiving over a transmission medium (e.g. the air). Alternatively, in the case of a wireless connection, the interface circuitry 14 may include means (e.g. a connector or plug) to enable the interface circuitry 14 to be connected to one or more suitable antennas external to the monitoring apparatus 2 for transmitting/receiving over a transmission medium (e.g. the air). The interface circuitry 14 is connected to the processing unit 10.

In general, the monitoring apparatus 2 can be any type of electronic device or computing device. In some implementations, for example as shown in Fig. 1, the monitoring apparatus 2 is an apparatus that is carried or worn by the user. For example, the monitoring apparatus 2 can be, or be part of, a laptop, a tablet, a smartphone, a smartwatch, etc., or be integrated into an item of clothing (e.g. a shirt, trousers, a coat, etc.) or be in the form of a wearable accessory (e.g. a wrist band, a pendant (including a Personal Help Button (PHB) that has a button that a user can push to summon help in an emergency), a necklace, a chest band, etc.). In other implementations, the monitoring apparatus 2 is an apparatus that is present or used in the home or care environment of the user. For example, the monitoring apparatus 2 can be, or be part of, a laptop, a tablet, a smartphone or a computer. In other implementations, the monitoring apparatus 2 is an apparatus that is remote from the user, and remote from the home or care environment of the user. For example, the monitoring apparatus 2 can be a server, for example a server in a data centre (also referred to as being 'in the cloud').

In order to monitor the user, the system 4 further comprises a movement sensor that is associated with the user, and that is for measuring the movements of the user. In some implementations, the movement sensor can be carried or worn by the user. In other implementations, the movement sensor can be remote from the user (i.e. not carried or worn by the user) and can observe the user, with the movements of the user being derived from the observations.

As noted above, in the implementation illustrated in Fig. 1, the monitoring apparatus 2 is in a form that can be carried or worn by the user, and a movement sensor 16 (also referred to herein as a 'first' movement sensor 16) is provided as part of the monitoring apparatus 2. The first movement sensor 16 is connected or coupled to the processing unit 10 (and/or to the memory unit 12).

Thus, the first movement sensor 16 measures the movements of the monitoring apparatus 2 (and thus the movements of the user, or a part of the body of the user, when the monitoring apparatus 2 is being worn or carried by the user).

It will be appreciated that although the first movement sensor 16 is shown as part of the monitoring apparatus 2 in Fig. 1, the first movement sensor 16 may be separate from the part of the monitoring apparatus 2 that includes the processing unit 10 (for example in a separate housing or body), and the first movement sensor 16 may be connected using a wired connection or wirelessly to the rest of the monitoring apparatus 2, including the processing unit 10 (e.g.

via the interface circuitry 14). For example the first movement sensor 16 may be part of a smart watch or pendant (including a PHB), and the processing unit 10 can be part of a smart phone to which the smart watch or pendant is paired.

In alternative implementations to that shown in Fig. 1, for example where the monitoring apparatus 2 is not in a form that can be carried or worn by the user, a movement sensor (which, for simplicity, is also referred to as first movement sensor 16) is provided in the system 4, but separate from the monitoring apparatus 2, and the first movement sensor 16 can be provided in a housing or body that can be worn or carried by the user (e.g. in the form of a pendant (including a PHB), necklace, watch, bracelet, wrist band, chest band or chest strap, etc.). In that case, the first movement sensor 16 may have interface circuitry associated therewith that enables the communication of the movement measurements to the processing unit 10 in the monitoring apparatus 2.

Regardless of the implementation of the monitoring apparatus 2 and the location of the first movement sensor 16 in the system 4, the first movement sensor 16 can generate a measurement signal that contains a plurality of movement measurement samples representing the movements of the user at a plurality of time instants.

Where the first movement sensor 16 is carried or worn by the user, the first movement sensor 16 may be an accelerometer that measures accelerations, and that provides a measurement signal indicating the accelerations measured in three dimensions. The first movement sensor 16 may alternatively be a gyroscope, an air pressure sensor or a magnetometer. Alternatively, the movements of the user can be measured using two or more movement sensors, with each movement sensor being any one of an accelerometer, gyroscope, air pressure sensor and magnetometer. Those skilled in the art will be aware of other types of movement sensor that can be used to monitor the movements of the user.

In implementations where the movement sensor can be remote from the user (i.e. not carried or worn by the user), the first movement sensor 16 can be a camera (or other type of imaging device), and the images of the user can be processed to extract the movements of the user (including the type of movements). Alternatively the first movement sensor 16 can be a motion sensor, such as a passive infrared (IR) sensor, that can detect motion in a sensing region of the passive IR sensor. Those skilled in the art will be aware of other types of movement sensor that can be used to remotely monitor the movements of the user.

The monitoring apparatus 2 may also comprise a user interface 18 that includes one or more components that enables a user of monitoring apparatus 2 to input information, data and/or commands into the monitoring apparatus 2, and/or enables the monitoring apparatus 2 to output information or data to the user of the monitoring apparatus 2. For example, the user interface 18 can be used to provide the alert to the user that they should use an AT device. Alternatively a user interface elsewhere in the system 4 (e.g. a user interface associated with an AT device or with another device used, carried or nearby the user) can be used to provide the alert. The user interface 18 can comprise any suitable input component(s), including but not limited to a keyboard, keypad, one or more buttons, switches or dials, a mouse, a track pad, a touchscreen, a stylus, a camera, a microphone, etc., and the user interface 18 can comprise any suitable output component(s), including but not limited to a display screen, one or more lights or light elements, one or more loudspeakers, a vibrating element, etc.

It will be appreciated that a practical implementation of a monitoring apparatus 2 may include additional components to those shown in Fig. 1. For example the monitoring apparatus 2 may also include a power supply, such as a battery, or components for enabling the monitoring apparatus 2 to be connected to a mains power supply.

As noted above, the system 4 includes a first monitoring device 6 that is associated with the first AT device 8 and that is used to measure the movements of the first AT device 8. The first monitoring device 6 includes a respective movement sensor 20 (referred to herein as a 'second' movement sensor 20) for measuring the movements of the first monitoring device 6 (and thus the movements of the first AT device 8), and respective interface circuitry 22 for enabling the movement measurements or processing results derived from the movement measurements to be provided to the monitoring apparatus 2. The interface circuitry 22 may be similar in functionality to the interface circuitry 14 in the monitoring apparatus 2, and thus may be used to establish a direct connection or an indirect connection (e.g. via the Internet) to the monitoring apparatus 2 via any desirable wired or wireless communication protocol.

The second movement sensor 20 may be similar to the first movement sensor 16 in the monitoring apparatus 2, and thus, for example, the second movement sensor 20 may be an accelerometer, gyroscope, an air pressure sensor or magnetometer, or a sensor (e.g. a camera or passive IR sensor) that remotely monitors or observes the first AT device 8. Those skilled in the art will be aware of other types of movement sensor that can be used to monitor the movements of the first AT device 8.

In some cases the first monitoring device 6 may also include a processing unit (not shown in Fig. 1) that may be used to perform some processing of the movement measurements to, for example, filter the movement measurements for draft and/or noise, or detect whether the first AT device 8 is/has been moving. In that case, the results of that processing can be provided to the monitoring apparatus 2 by the interface circuitry 22 (in addition to or instead of the movement measurements by the second movement sensor 20 in the first monitoring device 6).

In some embodiments or implementations, the system 4 may include more than one AT device (of the same or different types) that the user can use at any particular time to improve or assist their mobility. For example, a user may have both a walking frame and a walking stick. As another example, a user may have a walking frame located on an upper floor of their house for use upstairs and another walking frame located on a lower floor of their house for use downstairs and outside. In that case, the movement of each AT device might be monitored, in which case respective monitoring devices can be provided for monitoring the movements of each AT device. Thus, Fig. 1 shows an optional second AT device 24 with a second monitoring device 26. The second monitoring device 26 is associated with the second AT device 24, and the second monitoring device 26 is used to measure the movements of the second AT device 24. The second AT device 24 may be the same type of AT device as the first AT device 8, or it may be a different type of AT device. The second monitoring device 26 may be implemented in the same way as the first monitoring device 6 described above, and for example can include a respective movement sensor 28 (which is referred to herein as a 'third' movement sensor 28) and respective interface circuitry 30 for communicating the movement measurements (and/or information/data/measurements derived therefrom) to the monitoring apparatus 2.

Briefly, according to exemplary embodiments of the invention, the processing unit 10 in the monitoring apparatus 2 processes the measurements of the movements of the user and the measurements of the movements of the first AT device 8 to determine if the user is using the first AT device 8 while the user is walking. In particular, the measurements of the movements of the user are processed to determine if the user is walking, and if the user is found to be walking, the processing unit 10 determines a correlation measure representing a correlation (or amount of correlation) between the walking movements of the user and the movements of the first AT device 8. An alert (for the user to use an AT device) can be initiated when the correlation measure indicates that the movements of the user and the movements of the first AT device 8 are not correlated. Since the need for the alert is determined using an evaluation of movement measurements (which can typically be obtained and shared in a low-power and unobtrusive way), embodiments of the invention provide an improved way of monitoring a user and providing an alert to the user that they should use an AT device.

In some embodiments, the correlation measure is used to set or update a state indication for the user. The state indication can have a state referred to as a 'distant' state, and the state indication can be set or updated to the 'distant' state after determining a correlation measure that indicates that the walking movements of the user and the movements of the first AT device 8 are not correlated. The state indication may also have a state referred to as a 'near' state, and the state indication can be set or updated to the 'near' state when the correlation measure indicates that the walking movements of the user and the movements of the first AT device 8 are correlated. In some embodiments, the alert can be initiated if it is determined that the movements are not correlated, and, before updating the state indication in response to the correlation measure, the state indication is the 'near' state. In alternative embodiments, the alert can be initiated if it is determined that the movements are not correlated and the state indication transitions to the 'distant' state. In other alternative embodiments, the alert can be initiated if it is determined that the movements are not correlated (which can also be expressed as initiating an alert if the state indication is the distant state).

In more detail, according to various embodiments, the measure of correlation between the movements of the user and the first AT device 8 (which represents the correlation between the movements or movement/activity patterns of the user and the first AT device 8) is used to traverse a state model that indicates whether the user is near to the first AT device 8. The state indication has at least two possible states, a 'near' state which is reached when there is correlation between the movements of the user when walking and the movements of the first AT device 8 and a 'distant' state that is reached when the movements of the user indicate that the user is walking, but the movements of the first AT device 8 indicate little or no correlation. It will be appreciated that, for there to be low correlation, the first AT device 8 may be stationary (i.e. motionless) or relatively stationary, or the first AT device 8 may be moving in a different way (for example it is being used by a different user).

In some embodiments, an alert for the user to use an AT device is initiated whenever the user is walking and the correlation measure indicates that the first AT device 8 is not moving with the user (although it will be appreciated that an alert may not be initiated continuously while the user is walking). In other embodiments, an alert may only be initiated when the state indication transitions (i.e. changes) from the 'near' state to the 'distant' state (which is caused by the correlation measure indicating that the user is not using the first AT device 8). These embodiments have the benefit of providing an alert when the user is more likely to be close to the first AT device 8 (since the state indication has only just changed from the 'near' state). These embodiments can also reduce the overall amount of alerts presented to the user or reduce the frequency with which alerts are presented.

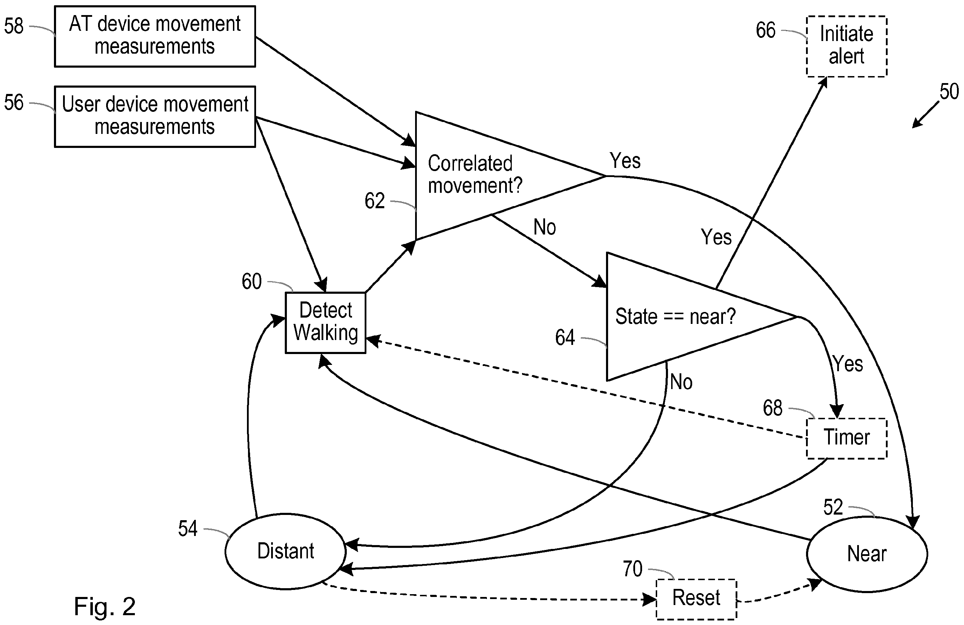

A state model 50 according to an exemplary embodiment is shown in Fig. 2. The state model 50 includes two states, a near state 52 and a distant state 54. Generally, the near state 52 occurs when the user is walking and the movements of the first AT device 8 are correlated with the user's movements. Generally, the distant state 54 occurs when the user is walking and the movements of the first AT device 8 are not correlated with the user's movements. The inputs to the state model 50 are the measurements of the movements of the user (referred to as user movement measurements 56) and the measurements of the movements of the first AT device 8 (referred to as AT device movement measurements 58).

The user movement measurements 56 are processed to determine if the user is walking (block 60). Techniques for the processing of movement measurements to detect walking (particularly movement measurements obtained from a movement sensor such as an accelerometer, gyroscope or magnetometer) are known in the art, and further details are not provided herein. It will be appreciated that, as used herein, 'walking' refers to the user moving with a stepping motion, and thus 'walking' includes ambulating, traversing, stepping (including going up and/or down stairs) and striding.

If walking is detected, the process in the state model 50 moves to block 62 where it is determined if there is correlation between the movements of the user and the movements of the first AT device 8. Thus, block 62 receives the user movement measurements 56 and the AT device movement measurements 58 and determines a measure of correlation.

If the correlation measure indicates that there is correlation (e.g. if the correlation measure exceeds a correlation threshold), then the near state 52 can be set. The near state 52 can be set as the correlation measure indicates that the user and first AT device 8 are moving together (e.g. the user is using the first AT device 8). The state model 50 then loops back to the block 60 where new (further) user movement measurements 56 are processed to determine if the user is walking.

If the correlation measure indicates that there is no correlation or insufficient correlation (e.g. if the correlation measure is below the correlation threshold), then the state indication is checked (block 64).

If the current state is the near state 52, then the state model 50 suggests that the first AT device 8 is near to the user and the user can easily pick up or use the first AT device 8. In this case, an alert can be initiated (block 66). The alert can remind or advise the user to use an AT device. It will be appreciated that the alert can remind the user to use a specific AT device, for example the first AT device 8, or the alert can just remind the user to use any AT device. The alert may be provided in any suitable form. For example the alert can be provided in a visual form (e.g. a light, a displayed message, etc.), an audible form (e.g. a beep, buzz, verbal message, etc.), a tactile form (e.g. a vibration, etc.), etc. The alert may be output to the user via the user interface 18 in the monitoring apparatus 2, and/or it may be output by a user interface associated with the first AT device 8 (e.g. a light on the first AT device 8 may flash, and/or a beep or buzz may sound from a loudspeaker on the first AT device 8). If at the check in block 64 the current state is the distant state 54, then no alert/reminder is issued (in this exemplary embodiment).

Regardless of the outcome of the check of the state indication in block 64, the distant state 54 is set as the state indication, as the user has been found to be walking and the movements of the first AT device 8 are not consistent (correlated) with the first AT device 8 being used by the user. The distant state 54 may be set once the check in block 64 is complete (or even on determining the 'no' outcome of the correlated movement check in block 62). Optionally, if the state indication check in block 64 identifies that the current state is the near state 52 (and therefore an alert is initiated in block 66), the setting of the state indication as the distant state 54 may be delayed until a timer expires (shown as optional block 68).

In addition, the initiation of the alert in block 66 may be delayed (e.g. by the same or a smaller amount of time as the delay in the setting of the state indication to the distant state 54) until at least one further check is performed of whether the movement of the user is correlated with the movement of the first AT device 8. This delay of the alert is not shown in Fig. 2. However, delaying the initiation of the alert in this way can allow time for the user to start walking and to shortly return to pick up (or otherwise start using) the first AT device 8, which will be recognised by a subsequent correlation check in block 62. If the subsequent correlation check in block 62 indicates that the movements are still not correlated (or sufficiently correlated), then an alert can be initiated (since the timer 68 means that the state will still be the near state 52).

After setting the state indication as the distant state 54, the model 50 loops back to 'detect walking' block 60.

The checking of the state indication in block 64 and initiating an alert only if the state is currently the near state 52 means that reminders/alerts may only be issued when the user is potentially near to the first AT device 8 and can easily/quickly start using it. This can help to suppress reminders/alerts when the user is far from the first AT device 8 (i.e. when the state indication is the distant state 52). However, in some embodiments, the check of the state indication in block 64 after finding that the correlation measure indicates that there is no correlation or insufficient correlation can be omitted. In these embodiments, after finding that there is no correlation or insufficient correlation, the alert can be initiated (block 66) and the state set to the distant state 54 (optionally after the timer (block 68) expires).

In implementations where the current state indication check in block 64 is used, it will be appreciated that alerts will not be initiated where the current state is the distant state 54 and the user starts walking. However, it may be the case that the user and the first AT device 8 were (correctly) distant from each other, but another person has given the first AT device 8 to the user or moved the first AT device 8 close to the user while, for example, the user is not walking (e.g. the user is sitting in a chair). In that case, it would be useful if the state model 50 triggered an alert to the user if they start walking but do not use the first AT device 8. Therefore, the state model 50 can be provided with an optional 'reset' step 70 that can be manually applied or triggered at any time by the user or another person that sets or resets the state indication to the near state 52. As an example, the reset can be triggered by pressing a button on the monitoring apparatus 2 or on the first monitoring device 6. As another example, when the monitoring apparatus 2 is in the form of a PHB device, the reset can be triggered by a particular sequence of button presses on the PHB device.

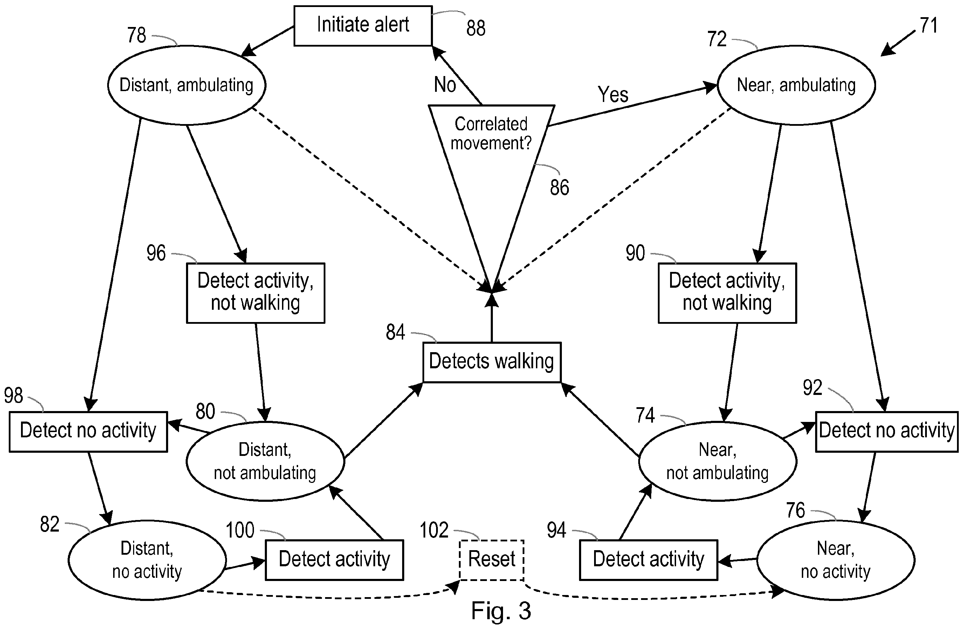

Fig. 3 shows a state model 71 according to another embodiment. This state model 71 includes six states, which are broadly grouped into near states and distant states. Within each group, there is a state representing walking (ambulating), the user being active but not walking, and the user being inactive. Thus, Fig. 3 shows a 'near, ambulating' state 72, a 'near, not ambulating' state 74, a 'near, no activity' state 76, a 'distant, ambulating' state 78, a 'distant, not ambulating' state 80 and a 'distant, no activity' state 82. Generally, the near states occur after detecting that the user has been walking and the movements of the first AT device 8 were correlated with the user's movements. Generally, the distant states occur after detecting that the user is walking and the movements of the first AT device 8 are not correlated with the user's movements. Although not shown in Fig. 3, the inputs to the state model 71 are the measurements of the movements of the user and the measurements of the movements of the first AT device 8.

Several detection blocks are shown in Fig. 3 which operate on the user movement measurements to detect whether the user is walking, active but not walking, or inactive, depending on the position of the detection block in the state model 71. A positive detection of the relevant state of the user by any of the blocks results in a change of the state indication for the user. Thus the type of detection performed at each detection block depends on its position in the state model 71.

A walking detection block 84 is provided that links the 'near, not ambulating' state 74 and the 'distant, not ambulating' state 80 to the 'near, ambulating' state 72 or the 'distant, ambulating' state 78, depending on the outcome of a check on whether the movement of the user is correlated with the movement of the first AT device 8 (shown as block 86). The walking detection block 84 is performed when the state indication is 'near, not ambulating' state 74 or 'distant, not ambulating' state 80. In some implementations, walking detection block 84 can also be performed when the state indication is 'near, ambulating' state 72 or 'distant, ambulating' state 78. In this case, the walking detection block 84 may be performed after the state indication has remained as 'near, ambulating' state 72 or 'distant, ambulating' state 78 for some time.

Block 86 operates in the same way as block 62 in Fig. 2. If correlation (or sufficient correlation) is found between the user movements and the movements of the first AT device 8, then the state indication is set to the 'near, ambulating' state 72. If no correlation (or insufficient correlation) is found between the user movements and the movements of the first AT device 8, then the state indication is set to the 'distant, ambulating' state 78.

In addition, if no correlation (or insufficient correlation) is found, then an alert is initiated to the user to remind them to use an AT device (block 88). The initiation of the alert in block 88 can be the same as or similar to the initiation of the alert according to block 66 of Fig. 2. Unlike in Fig. 2, the state model 71 in Fig. 3 does not require the current state indication to be the near state (or a near state) in order for an alert to be issued. In this case, an alert/reminder can be issued or triggered regardless of the current state of the user. However, it will be appreciated that in alternative implementations, the state model 71 can include a check similar to that shown in block 64 of Fig. 2.

The other detection blocks include:

- a detection block 90 for when the state indication is 'near, ambulating' state 72 for detecting if the user is active but not walking, with a positive detection of active but not walking causing the state indication to change to 'near, not ambulating' state 74;
- a detection block 92 for when the state indication is 'near, ambulating' state 72 or 'near, not ambulating' state 74 for detecting if the user is active, with a negative detection of activity by the user (i.e. the user is inactive) causing the state indication to change to 'near, no activity' state 76;
- a detection block 94 for when the state indication is 'near, no activity' state 76 for detecting if the user is now active, with a positive detection of the user now being active causing the state indication to change to 'near, not ambulating' state 74;
- a detection block 96 for when the state indication is 'distant, ambulating' state 78 for detecting if the user is active but not walking, with a positive detection of active but no positive detection of walking causing the state indication to change to 'distant, not ambulating' state 80;
- a detection block 98 for when the state indication is 'distant, ambulating' state 78 or 'distant, not ambulating' state 80 for detecting if the user is active, with a negative detection of activity by the user (i.e. the user is inactive) causing the state indication to change to 'distant, no activity' state 82; and
- a detection block 100 for when the state indication is 'distant, no activity' state 82 for detecting if the user is now active, with a positive detection of the user now being active causing the state indication to change to 'distant, not ambulating' state 80.

It will be appreciated that there are similarities between the functions of several of the detection blocks mentioned above, and thus some detection blocks in Fig. 3 can be implemented using the same function/algorithm/software (e.g. the processing/analysis performed by detection blocks 92, 98 is the same and could be implemented in practice by a single function/algorithm/software).

As in the state model 50 shown in Fig. 2, the state model 71 can include an optional reset block 102 that corresponds to the reset block 70 in Fig. 2.

Activity by the user can be detected by processing the user movement measurements in a number of different ways. In one example, activity can be detected as a certain level (e.g. above a threshold) of variance in the movement measurements. Likewise, 'no activity' can be detected where the certain level is below the threshold. Those skilled in the art will be aware of other ways to determine activity are known in the art.

It will be appreciated that alternative implementations of the state model 71 shown in Fig. 3 are possible. For example, the 'no activity' and 'not ambulating' near states (74 and 76) can be combined or considered as a single state and the 'no activity' and 'not ambulating' distant states (80 and 82) can be combined or considered as a single state.

Similar to the state model 50 in Fig. 2, a timer can be used to delay transitions to a new state indication. A timer can be used or applied for any of the state transitions shown in Fig. 3. In some implementations, where a timer is used, the prior detection block can continue detecting the state of the user (e.g. walking, active but not walking, or inactive) while the timer is running, which can mean that the state indication only transitions once the state of the user has been detected for a required amount of time. This can provide a more stable and robust decision whether to transition to the next state.

In some implementations (which can also apply to the timer in Fig. 2), the length or duration of the timer can be predetermined and set to the same value regardless of the user. However, in other implementations (which can also apply to the timer in Fig. 2), the length or duration of the timer can be adapted to the particular user.

In some implementations (which can also apply to the state model 50 in Fig. 2), the thresholds used to detect activity/no activity or walking/not walking can be predetermined and set to the same value(s) regardless of the user. However, in other implementations (which can also apply to the state model 50 in Fig. 2), the thresholds used to detect activity/no activity or walking/not walking can be adapted to the particular user. For example, the user may have an unusual or non-standard gait, and a threshold used for walking detection can be adapted to enable walking to be more reliably detected for that user.

It will be appreciated from Figs. 2 and 3 that the AT device movement measurements are only processed once the user has been found to be walking. In this case, it is possible to reduce the power consumption of the system 4 by deactivating the second movement sensor 20 and/or deactivating the communication of movement measurements from the first monitoring device 6 to the monitoring apparatus 2 until it is detected that the user is walking. In that case, once walking is detected in detection block 60 or detection block 84, a control signal can be sent to the first monitoring device 6 to start the measuring of the movements of the first AT device 8 and/or to start the communication (e.g. transmission) of the movement measurements from the first monitoring device 6 to the monitoring apparatus 2. Alternatively (or in addition), the first monitoring device 6 can deactivate or enter a 'sleep' mode if the first monitoring device 6 (and thus the first AT device 8) is not moving or being moved. For example, if the second movement sensor 20 is an accelerometer, the first monitoring device 6 may enter a sleep mode if the accelerometer does not detect any motion (or any significant motion), and the accelerometer can output a 'wake-up' signal to other processing electronics in the first monitoring device 6 when the accelerometer detects motion.

As noted above, in some embodiments the system 4 can include multiple AT devices that the user can use to assist their mobility (e.g. the user may have a walking stick and a walking frame), and the movements of each of the AT devices can be monitored as described above. In that case, the state model 50 in Fig. 2 and the state model 71 in Fig. 3 can be adapted for use with the multiple AT devices by performing a separate check for correlation between the user movement measurements and the movement measurements of each AT device in block 62 of state model 50 and block 84 of state model 71. That is, in block 62 or 84, a first correlation measure is determined from the user movement measurements and the movement measurements for the first AT device 8, a second correlation measure is determined from the user movement measurements and the movement measurements for a second AT device, and so on, for each of the monitored AT devices in the system 4. If any of the determined correlation measures indicates that there is correlation (e.g. if any of correlation measures exceeds a correlation threshold), then the near state 52 or 'near, ambulating' state 72 can be set, and no alert or reminder needs to be initiated as the user is using one of the monitored AT devices. If none of the determined correlation measures indicates that there is correlation (e.g. if none of the correlation measures exceeds a correlation threshold), then the distant state 54 or 'distant, ambulating' state 78 can be set, and an alert or reminder may need to be initiated as the user is not using any of the monitored AT devices (it will be appreciated that the initiating of the alert on determining that the user is in a 'distant' state may be subject to the current state indication check in block 64 of state model 50).

In some embodiments, the system 4 may include more than one device (and in particular more than one movement sensor) that can monitor the movements of the user. For example, the user may carry or wear a PHB pendant that includes a movement sensor, and the user may also carry a smartphone that includes another movement sensor. In this case, the measurements from each sensor can be processed together to determine if the user is walking and/or if the user movements are correlated with the AT device movement measurements.

Alternatively, the user movement measurements from each of the user devices (PHB, smartphone, etc.) can be processed separately to determine if the user is walking (or active/inactive). The user can be determined to be walking if the user movement measurements from at least one of the user devices indicates walking. In that case, the correlation check block 62/86 can determine a correlation between the user movement measurements that indicate walking and the AT device movement measurements (this means that the user movement measurements from the other user device(s) are not used in the correlation check block). If the user movement measurements from several of the user devices indicate walking, then a separate correlation measure can be determined for the user movement measurements from each user device and the AT device movement measurements. If at least one of these correlation measures indicates that there is correlation, then no alert is required. However, if none of these correlation measures indicates that there is correlation, then an alert can be initiated (subject to the current state check in block 64, if required).

In a variation to the state model 71 shown in Fig. 3, the check for correlation between the user movement measurements and the AT device movement measurements in block 86 may only be performed at the start of a walking movement (i.e. rather than performing the check whenever walking is (currently) detected). In this case, the 'near, not ambulating' state 74 can be understood as a 'near, standing' state and the 'distant, not ambulating' state 80 can be understood as a 'distant, standing' state, and the detection blocks 94, 100 that cause the transition to the respective 'standing' states can detect whether the user is now in a standing posture (e.g. the detection blocks 94, 100 can detect if the user has performed a sit-to-stand movement, or has got out of bed). Likewise, the 'near, no activity' state 76 and the 'distant, no activity' state 82 can be understood as a 'near, sitting/lying' state and a 'distant, sitting/lying' state respectively, with the detection blocks 92, 98 that cause the transition to the respective 'sitting/lying' states can detect if the user has performed a stand-to-sit movement or is in a sitting or lying posture.

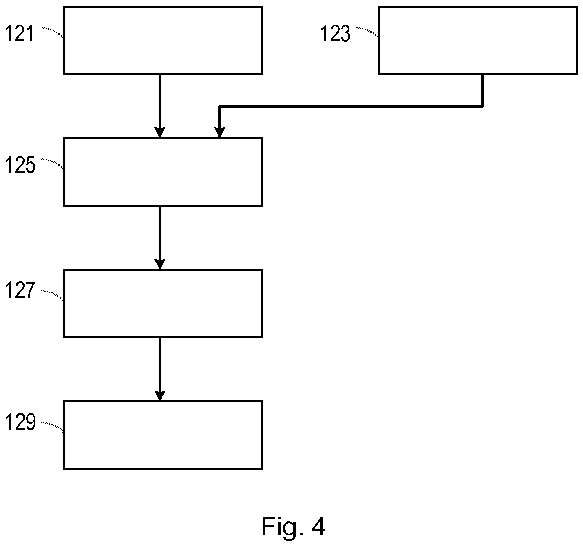

The flow chart in Fig. 4 illustrates a method of monitoring a user according to the techniques described herein. One or more of the steps of the method can be performed by the processing unit 10 in the monitoring apparatus 2, in conjunction with any of the memory unit 12, interface circuitry 14 and user interface 18 as appropriate. The processing unit 10 may perform the one or more steps in response to executing computer program code, that can be stored on a computer readable medium, such as, for example, the memory unit 12.

In step 121, measurements of movements of the user are received. These movement measurements are also referred to herein as 'first movement measurements'. These movement measurements are obtained by the first movement sensor 16 which is associated with the user. As noted above, in some embodiments, the first movement sensor 16 is worn or carried by the user. The measurements of movements may be received in real-time or near real-time, or they may have been temporarily stored in memory unit 12 and are retrieved from the memory unit 12 in step 121.

In step 123, measurements of the movements of first AT device 8 are received. These movement measurements are also referred to herein as 'second movement measurements'. These measurements of movements are obtained by a movement sensor that is associated with the first AT device 8. The second movement measurements may be received in real-time or near real-time, or they may have been temporarily stored in memory unit 12 and are retrieved from the memory unit 12 in step 123.

Step 123 may occur at the same time as step 121, or steps 121 and 123 may occur at different times, with either step 121 or 123 occurring before the other. In some embodiments, step 123 may occur after step 125. In some embodiments, step 123 may only occur after determining in step 125 that the user is walking.

In step 125, the first movement measurements are processed to determine if the user is walking. As noted above with regard to the description of the state models 50 and 71, walking can be detected from the movement measurements using techniques known in the art. Also as noted above, 'walking' refers to the user moving with a stepping motion, and thus 'walking' includes ambulating, traversing, stepping (including going up and/or down stairs) and striding.

If it is determined that the user is walking in step 125, then in step 127 a correlation measure is determined for the first movement measurements and the second movement measurements. The correlation measure represents the correlation between the movements of the user while the user is walking (as represented by the first movement measurements) and the movements of the first AT device 8 (as represented by the second movement measurements).

In general, if the user is using the first AT device 8 while the user is walking, there should be a (sufficient) correlation between the movements of the user and the movements of the first AT device 8. Movement/activity of a first AT device 8 should match or correlate with movement by the user, particularly for a period of time (e.g. 3 or 4 seconds) before a positive indication of correlation can be determined.

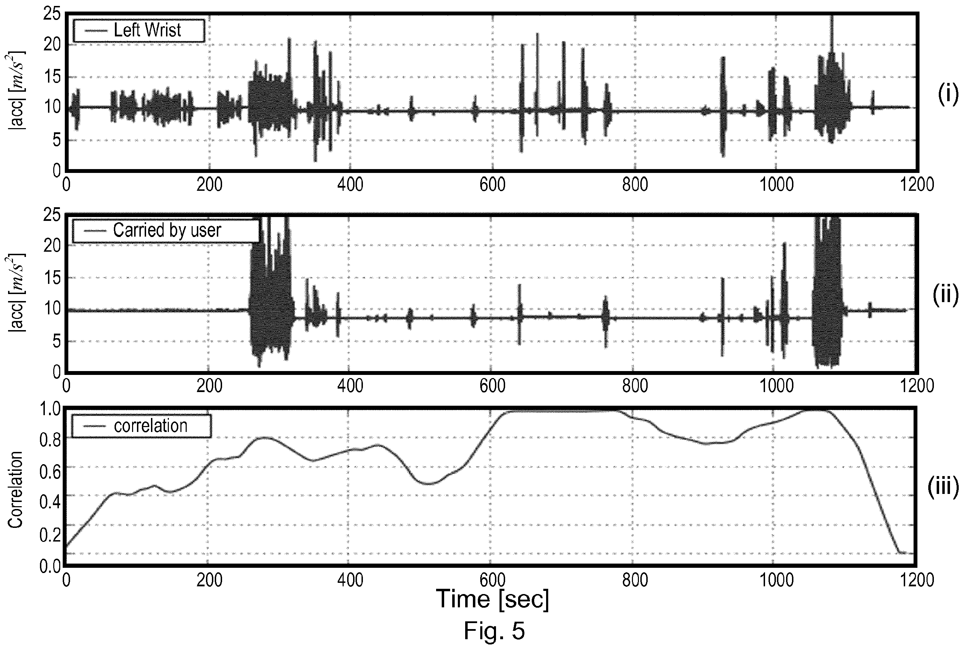

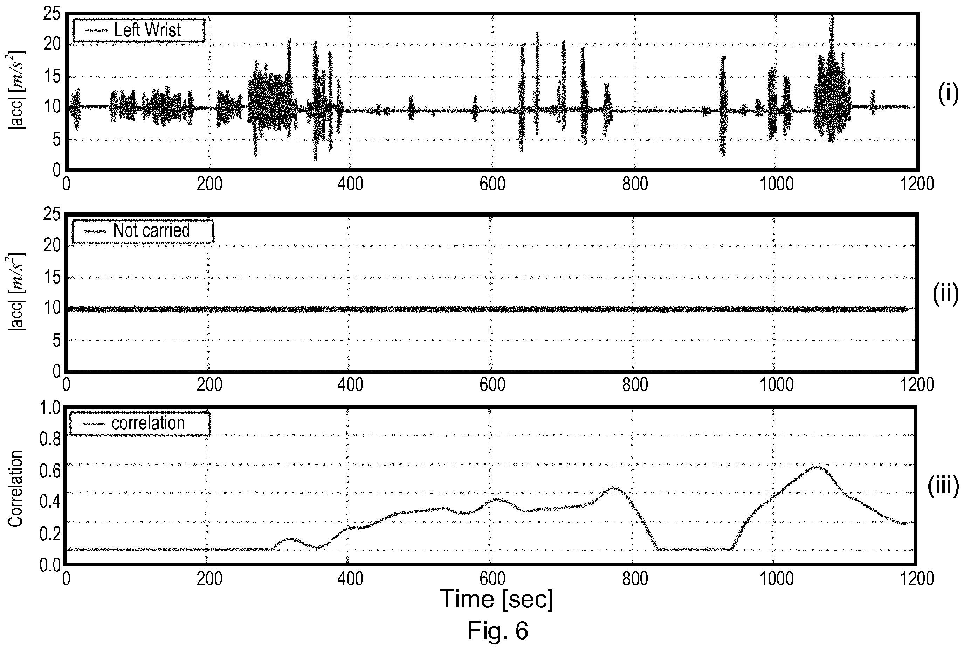

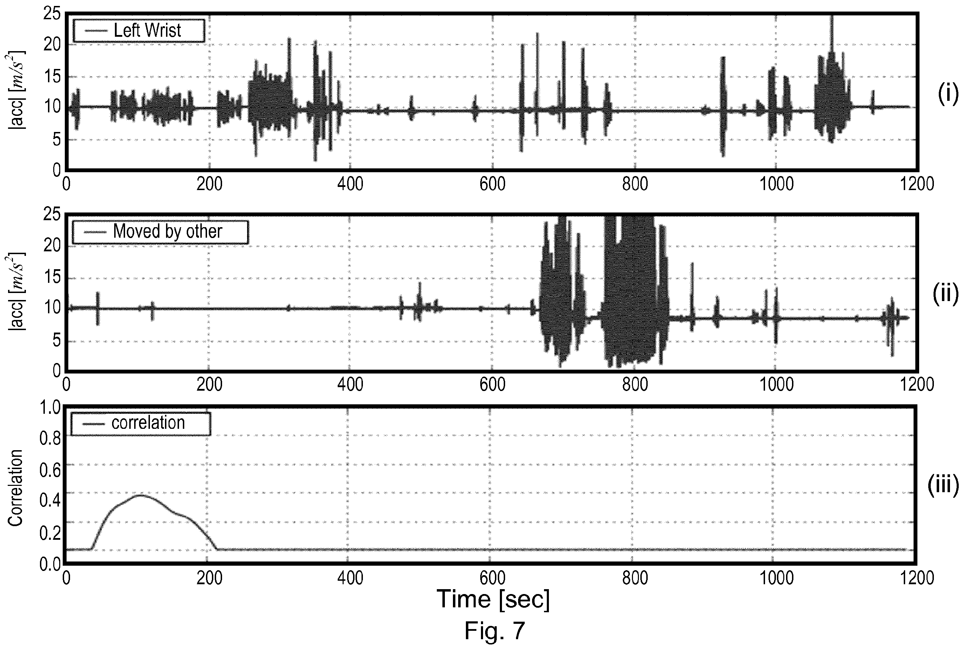

An embodiment of step 127 in which movements of the user are compared to movements of the first AT device 8 to determine a measure of correlation between the measurements is described below with reference to Figs. 5, 6 and 7. Figs. 5(i), 6(i) and 7(i) each show an exemplary measurement signal that is the norm of a set of three dimensional acceleration measurements for a time period of 1200 seconds obtained from an accelerometer 16 that is being worn on the left wrist of the user (and thus Figs. 5(i), 6(i) and 7(i) represent the movements of the user). Figs. 5(ii), 6(ii) and 7(ii) each show an exemplary measurement signal that is the norm of a set of three dimensional acceleration measurements for the same time period as Figs. 5(i), 6(i) and 7(i) for an accelerometer 20 in a first AT device 8. Figs. 5(ii), 6(ii) and 7(ii) represent different states of motion for the accelerometer 20/first AT device 8. In Fig. 5(ii), the first AT device 8 is being carried or used by the user, in Fig. 6(ii), the first AT device 8 is not being carried or used by the user or any other person (and so the normed acceleration measurements just indicate the norm of acceleration due to gravity, i.e. 9.81 ms$^{-2}$ (with measurement noise by the second movement sensor 20), and in Fig. 7(ii), the first AT device 8 is being carried or used by a different person to the user wearing or carrying the first movement sensor 16. Figs. 5(iii), 6(iii) and 7(iii) show a respective correlation signal derived from the two normed acceleration signals in each Figure. Briefly, it can be seen that the movements of the user and the first AT device 8 exhibit relatively high correlation in Fig. 5(iii) (i.e. correlation above 0.5) where the first AT device 8 is being used by the user, and is thus subject to largely the same movement patterns. The measurements exhibit much lower correlation in Fig. 6(iii) (i.e. correlation below 0.5) where the first AT device 8 is not moving. Finally, the measurements again exhibit low correlation in Fig. 7(iii) (i.e. correlation below 0.5) where the first AT device 8 is being moved by a different person to the user.

In some embodiments, where the movement measurements are measurements of acceleration, the norm of the acceleration measurements for each of the user and the first AT device 8 can be determined. This norm can be seen as a measure of the activity of the user/first AT device 8 (as appropriate).

In a first step to determine if the movements of the user and the first AT device 8 are correlated, the acceleration norm signals can be low-pass filtered (LPF), for example using a moving average filter with a half-window size of 20 seconds (although those skilled in the art will appreciate that other half-window sizes can be used).

In a second step, the LPF signals can be correlated, for example using a sliding window with half size of 80 seconds (although again those skilled in the art will appreciate that other half-window sizes can be used). The correlation at a certain time instant (sample) k is given by:

$$cc[k] = sum((s_0[k_0{:}k_1]{-}mn_0) * (s_1[k_0{:}k_1]{-}mn_1))/sqrt(var(s_0) * var(s_1))$$

where $s_0[k_0{:}k_1]$ indicates the sample sequence from $k_0$ to $k_1$ of the signal (LPF of the norm of the acceleration) of the fall detection apparatus 2, $s_1$ likewise for the object 6, $k_0$ to $k_1$ span the (2*80 sec) window, centred around current sample k, i.e.:

$$k_0 = k - 80 \ sec$$

$$k_1 = k + 80 \ sec$$

mno and $mn_1$ represent the mean over that span of signal so and $s_1$, respectively, var indicates the variance, and sqrt the square root operator.

In a third step, the obtained series of cc (correlation) values are preferably smoothed (e.g. using another low pass filter), for example using a half window of 60 seconds (although again those skilled in the art will appreciate that other half-window sizes can be used). Preferably, negative values are clipped to 0. In this way, the correlation, cc, ranges between 0 and 1.

The cc values can then be tested (compared) against a threshold, for example 0.5, although other values can be used if desired. Correlation values above the threshold indicate the first AT device 8 is carried or used by the user, and correlation values below the threshold indicate that it is not.

In step 129, an alert is initiated if the correlation measure indicates that the user is not using the first AT device 8. The alert can remind or advise the user to use any AT device or to use the first AT device 8. The alert can be issued to the user in a number of different forms, as outlined above with regard to the state models 50, 71 in Figs. 2 and 3.

The method in Fig. 4 can then repeat for subsequent movement measurements of the user.

In some embodiments, the step 127 of determining a measure of correlation between the measurements of the movements of the user and the first AT device 8 can be accompanied by, or preceded by, a check on the movement of the first AT device 8. In particular, this check can determine whether the first AT device 8 is moving/active, and if this check indicates that the first AT device 8 is not moving or active, then an alert can be initiated, without waiting for (or potentially without performing) the correlation check.

In some embodiments, the method further comprises a step of updating a state indication for the user and the first AT device 8 based on the correlation measure determined in step 127. This state indication is maintained until it subsequently needs to be updated or changed. The state indication has one of at least two states, and the correlation measure determined in step 127 is used to determine which state the user and the first AT device 8 are in, and/or used to determine when to transition from a state to another state.

The at least two states comprises a near state, and the state indication is updated to the near state if the correlation measure determined in step 129 indicates that the (walking) movements of the user are correlated with the movements of the first AT device 8. The near state thus corresponds to the near state 52 in the state model 50 of Fig. 2 or the 'near, ambulating' state 72 in the state model 71 of Fig. 3. The at least two states also comprises a distant state, and the state indication is updated to the distant state if the correlation measure determined in step 129 indicates that the (walking) movements of the user are not correlated with the movements of the first AT device 8. The distant state thus corresponds to the distant state 54 in the state model 50 of Fig. 2 or the 'distant, ambulating' state 72 in the state model 71 of Fig. 3. In some embodiments, the at least two states can also include any one or more of the other states shown in Figs 2 and 3, or the variations to those states described above.

If a state transition is required (e.g. the state indication is the 'near' state and the correlation measure indicates that the user is not using the first AT device 8 while walking), the state transition can occur as soon as the correlation measure is determined in step 127, or a timer or timeout can be used to delay the state transition, for example for a few seconds to allow time for the user to pick up the first AT device 8.

In some embodiments, the alert can be initiated in step 129 if it is determined that the movements are not correlated, and, before updating the state indication in response to the correlation measure, the state indication is the 'near' state.

In alternative embodiments, the alert can be initiated in step 129 if it is determined that the movements are not correlated and the state indication transitions to the 'distant' state.

In other alternative embodiments, the alert can be initiated in step 129 if the state indication is the distant state (which is the same as indicating that the alert is initiated if the movements are not correlated).

There is therefore provided an improved way of monitoring a user and providing an alert to a user that they should use an AT device.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

**Claims**

1. A monitoring apparatus (2) for monitoring a user, the monitoring apparatus (2) comprising:

    a processing unit (10) configured to:

        receive first movement measurements from a first movement sensor (16) that is associated with the user, the first movement measurements representing movements of the user;
        receive second movement measurements from a second movement sensor (20) that is associated with a first assistive technology, AT, device (8), the second movement measurements representing movements of the first AT device (8);
        process the first movement measurements to determine if the user is walking;
        if it is determined that the user is walking, determine a correlation measure for the first movement measurements and the second movement measurements, wherein the correlation measure represents the correlation between the movements of the user while the user is walking and the movements of the first AT device (8); and
        initiate an alert to the user to use an AT device if the determined correlation measure indicates that the movements of the user and the movements of the first AT device (8) are not correlated.

2. A monitoring apparatus (2) as claimed in claim 1, wherein the processing unit (10) is further configured to:

        update a state indication for the user and the first AT device (8) based on the determined correlation measure, wherein the state indication has one of at least two states, wherein the at least two states comprises a near state and a distant state, wherein the state indication is set to the near state if the determined correlation measure indicates that the movements of the user and the movements of the first AT device (8) are correlated, and wherein the state indication is set to the distant state if the determined correlation measure indicates that the movements of the user and the movements of the first AT device (8) are not correlated.

3. A monitoring apparatus (2) as claimed in claim 2, wherein the processing unit (10) is configured to:

initiate the alert to the user if the determined correlation measure indicates that the movements of the user and the movements of the first AT device (8) are not correlated, and, before updating the state indication based on the determined correlation measure, the state indication is the near state.

4. A monitoring apparatus (2) as claimed in claim 2, wherein the processing unit (10) is configured to:

initiate the alert to the user if the determined correlation measure indicates that the movements of the user and the movements of the first AT device (8) are not correlated and the state indication is updated to the distant state.

5. A monitoring apparatus (2) as claimed in any of claims 2-4, wherein the processing unit (10) is further configured to:

if the determined correlation measure indicates that the movements of the user and the movements of the first AT device (8) are not correlated, wait for the expiry of a timer before initiating the alert.

6. A monitoring apparatus (2) as claimed in any of claims 1-5, wherein the processing unit (10) is configured to receive the second movement measurements from the second movement sensor (20) after determining that the user is walking.

7. A monitoring apparatus (2) as claimed in any of claims 1-6, wherein the processing unit (10) is further configured to:

receive third movement measurements from a third movement sensor (28) that is associated with a second AT device (24), the third movement measurements representing movements of the second AT device (24);
if it is determined that the user is walking, determine a second correlation measure for the first movement measurements and the third movement measurements, wherein the second correlation measure represents the correlation between the movements of the user while the user is walking and the movements of the second AT device (24); and
wherein the processing unit (10) is configured to initiate the alert to the user if the correlation measure indicates that the movements of the user and the movements of the first AT device (8) are not correlated and the second correlation measure indicates that the movements of the user and the movements of the second AT device (24) are not correlated.

8. A method of monitoring a user, the method comprising:

receiving (121) first movement measurements from a first movement sensor that is associated with the user, the first movement measurements representing movements of the user;
receiving (123) second movement measurements from a second movement sensor that is associated with a first assistive technology, AT, device, the second movement measurements representing movements of the first AT device;
processing (125) the first movement measurements to determine if the user is walking;
if it is determined that the user is walking, determining (127) a correlation measure for the first movement measurements and the second movement measurements, wherein the correlation measure represents the correlation between the movements of the user while the user is walking and the movements of the first AT device; and
initiating (129) an alert to the user to use an AT device if the determined correlation measure indicates that the movements of the user and the movements of the first AT device are not correlated.

9. A method as claimed in claim 8, wherein the method further comprises:

updating a state indication for the user and the first AT device based on the determined correlation measure, wherein the state indication has one of at least two states, wherein the at least two states comprises a near state and a distant state, wherein the state indication is set to the near state if the determined correlation measure indicates that the movements of the user and the movements of the first AT device are correlated, and wherein the state indication is set to the distant state if the determined correlation measure indicates that the movements of the user and the movements of the first AT device are not correlated.

10. A method as claimed in claim 9, wherein the method comprises:

initiating (129) the alert to the user if the determined correlation measure indicates that the movements of the user and the movements of the first AT device are not correlated, and, before updating the state indication based on the determined correlation measure, the state indication is the near state.

11. A method as claimed in claim 9, wherein the method comprises:

initiating (129) the alert to the user if the determined correlation measure indicates that the movements of the user and the movements of the first AT device are not correlated and the state indication is updated to the distant state.

12. A method as claimed in any of claims 9-11, wherein the method further comprises:

if the determined correlation measure indicates that the movements of the user and the movements of the first AT device are not correlated, waiting for the expiry of a timer before initiating the alert.

13. A method as claimed in any of claims 8-12, wherein the step of receiving (123) the second movement measurements from the second movement sensor is performed after determining that the user is walking.

14. A method as claimed in any of claims 8-13, wherein the method further comprises:

receiving third movement measurements from a third movement sensor that is associated with a second AT device, the third movement measurements representing movements of the second AT device;
if it is determined that the user is walking, determining a second correlation measure for the first movement measurements and the third movement measurements, wherein the second correlation measure represents the correlation between the movements of the user while the user is walking and the movements of the second AT device; and
wherein the step of initiating the alert to the user comprises initiating the alert to the user if the correlation measure indicates that the movements of the user and the movements of the first AT device are not correlated and the second correlation measure indicates that the movements of the user and the movements of the second AT device are not correlated.

15. A computer program product comprising a computer readable medium having computer readable code embodied therein, the computer readable code being configured such that, on execution by a suitable computer or processor, the computer or processor is caused to perform the method of any of claims 8-14.

Fig. 1

EP 3 627 515 A1

Fig. 2

Fig. 3

121

123

125

127

129

Fig. 4

Fig. 5

Fig. 6

Fig. 7

Europäisches
Patentamt

European
Patent Office

Office européen
des brevets

# EUROPEAN SEARCH REPORT

Application Number

EP 18 19 5446

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2017/154514 A1 (CONDON HEATHER G [US]) 1 June 2017 (2017-06-01) * claims * | 1-15 | INV. G16H20/30 G08B21/02 G08B21/04 A61H3/00 A61B5/11 |
| Y | Thomas : Yen: "Alert System and Cadence Walker", , 1 October 2012 (2012-10-01), XP55561133, Retrieved from the Internet: URL:http://bmedesign.engr.wisc.edu/projects/file/?fid=2731 [retrieved on 2019-02-25] * the whole document * | 1-15 | |
| X | US 2012/184878 A1 (NAJAFI BIJAN [US] ET AL) 19 July 2012 (2012-07-19) | 1-6, 8-13,15 | |
| Y | * paragraphs [0012], [0016], [0019], [0020], [0034], [0041]; claims; figures 1,2,3 * | 1-15 | |
| X | US 2013/346021 A1 (STEVENS MARK B [US] ET AL) 26 December 2013 (2013-12-26) | 1-6, 8-13,15 | TECHNICAL FIELDS SEARCHED (IPC) |
| Y | * paragraphs [0034] - [0036], [0040], [0055]; claims; figures 5,6,7,8 * | 1-15 | G16H G08B A61H A61B |
| Y | WO 2011/004322 A1 (KONINKL PHILIPS ELECTRONICS NV [NL]; TEN KATE WARNER RUDOLPH THEOPHILE) 13 January 2011 (2011-01-13) * claims; figures 1,2,3 * | 1-15 | |
| Y | WO 2018/003910 A1 (NEC CORP [JP]) 4 January 2018 (2018-01-04) * claims; figures 4,6,9 * | 1-15 | |
| E | US 2018/333052 A1 (CHAMBERLAIN PETER THOMAS [US]) 22 November 2018 (2018-11-22) * paragraph [0041]; claims; figures 9,10 * | 1-15 | |

The present search report has been drawn up for all claims

| Place of search | Date of completion of the search | Examiner |
|---|---|---|
| Munich | 26 February 2019 | Vogt, Titus |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 3 627 515 A1**

## ANNEX TO THE EUROPEAN SEARCH REPORT
## ON EUROPEAN PATENT APPLICATION NO.

EP 18 19 5446

This annex lists the patent family members relating to the patent documents cited in the above-mentioned European search report.
The members are as contained in the European Patent Office EDP file on
The European Patent Office is in no way liable for these particulars which are merely given for the purpose of information.

26-02-2019

| Patent document cited in search report | | Publication date | Patent family member(s) | | Publication date |
|---|---|---|---|---|---|
| US 2017154514 | A1 | 01-06-2017 | NONE | | |
| US 2012184878 | A1 | 19-07-2012 | NONE | | |
| US 2013346021 | A1 | 26-12-2013 | NONE | | |
| WO 2011004322 | A1 | 13-01-2011 | AU | 2010269846 A1 | 01-03-2012 |
| | | | CN | 102469955 A | 23-05-2012 |
| | | | EP | 2451351 A1 | 16-05-2012 |
| | | | JP | 5647240 B2 | 24-12-2014 |
| | | | JP | 2012532652 A | 20-12-2012 |
| | | | RU | 2012104679 A | 20-08-2013 |
| | | | US | 2012095722 A1 | 19-04-2012 |
| | | | US | 2015226764 A1 | 13-08-2015 |
| | | | WO | 2011004322 A1 | 13-01-2011 |
| WO 2018003910 | A1 | 04-01-2018 | NONE | | |
| US 2018333052 | A1 | 22-11-2018 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2017154514 A